# EUROPEAN PATENT SPECIFICATION

(11) **EP 3 695 803 B1**
(45) Date of publication and mention of the grant of the patent: **05.05.2021**
(21) Application number: 19156856.7
(22) Date of filing: 13.02.2019
(51) Int. Cl.: A61C 8/02, C12N 5/0775, A61K 35/28, A61K 35/16

(54) **KIT AND METHOD FOR IMPROVED BONE REGENERATION**
KIT UND VERFAHREN FÜR VERBESSERTE KNOCHENREGENERATION
KIT ET PROCÉDÉ PERMETTANT D'AMÉLIORER LA RÉGÉNÉRATION OSSEUSE

(43) Date of publication of application: 19.08.2020
(73) Proprietor: Solakoglu, Önder, 22359 Hamburg (DE)
(72) Inventor: Solakoglu, Önder, 22359 Hamburg (DE)
(74) Representative: Glawe, Delfs, Moll

(56) References cited:
- HENK-JAN PRINS ET AL: "Bone Regeneration Using the Freshly Isolated Autologous Stromal Vascular Fraction of Adipose Tissue in Combination With Calcium Phosphate Ceramics : Bone Regeneration With Autologous Fat Stem Cells", STEM CELLS TRANSLATIONAL MEDICINE, vol. 5, no. 10, 7 July 2016 (2016-07-07), pages 1362-1374, XP055599996, US ISSN: 2157-6564, DOI: 10.5966/sctm.2015-0369
- PANAGIOTIS DRAGONAS ET AL: "Plasma rich in growth factors (PRGF) in intraoral bone grafting procedures: A systematic review", JOURNAL OF CRANIO-MAXILLO-FACIAL SURGERY, vol. 47, no. 3, 17 January 2019 (2019-01-17), pages 443-453, XP055599990, GB ISSN: 1010-5182, DOI: 10.1016/j.jcms.2019.01.012
- DEBORAH CHICHARRO ET AL: "Combined plasma rich in growth factors and adipose-derived mesenchymal stem cells promotes the cutaneous wound healing in rabbits", BMC VETERINARY RESEARCH, vol. 14, no. 1, 21 September 2018 (2018-09-21), XP055599993, DOI: 10.1186/s12917-018-1577-y
- ÖNDER SOLAKOGLU ET AL: "Improved guided bone regeneration by combined application of unmodified, fresh autologous adipose derived regenerative cells and plasma rich in growth factors: A first-in-human case report and literature review", WORLD JOURNAL OF STEM CELLS, vol. 11, no. 2, 26 February 2019 (2019-02-26), pages 124-146, XP055599934, CN ISSN: 1948-0210, DOI: 10.4252/wjsc.v11.i2.124

## Description

### FIELD OF THE INVENTION

Provided herein are kits and methods for improving bone regeneration, in particular guided bone regeneration (GBR) in oral surgery prior to implant placement, particularly in maxillary sinus augmentation (GBR-MSA) and in lateral alveolar ridge augmentation (LRA).

### BACKGROUND OF THE INVENTION

The main causes of tooth loss are periodontal disease and dental caries^{[1]}. In fact, in particular maxillary posterior partial or complete edentulism is one of the most common occurrences in dentistry. For example, 17.5% of the adult US population are missing all of their maxillary teeth. Replacing missing or lost teeth with osseointegrated dental implants has been highly successful in the treatment of single, partial, or complete edentulism^{[2]}. However, insufficient bone volume is a common problem occurring in the rehabilitation of the edentulous posterior maxilla with implant-supported prostheses^{[3-5]}. Both the presence of the maxillary sinus and atrophy of the alveolar process after tooth extraction or loss contribute to this problem. Therefore, it is often necessary to perform vertical alveolar ridge augmentation to enable implant placement and integration. A well-studied technique in this context is maxillary sinus membrane elevation with autologous bone or a variety of biomaterials (i.e., a form of guided bone regeneration in maxillary sinus augmentation; thereafter, "GBR-MSA")^{[3-6]}. However, for the following reasons there is a need for developing novel strategies for improving GBR-MSA.

First, autologous bone is considered to be the gold standard in GBR-MSA due to its osteogenic, osteoinductive, and osteoconductive properties including lack of immunogenicity^{[7,8]}. However, autologous bone grafts may show a number of disadvantages, such as increased operation time, donor site morbidity, post-operative discomfort, limitations in bone quantity and volume, unpredictable bone quality, reduced volume stability, and fast resorption rate^{[9-13]}. It may also be only effective under good recipient conditions. Furthermore, the intraoral amount of autologous bone is limited, and therefore extraoral donor sites are needed for larger defects. Extraoral donor sites like the iliac crest may lead to further discomfort for the patient and an even higher morbidity rate compared to intraoral donor sites.

Second, while allografts have osteogenic properties, their probable osteoinductive and osteoconductive functions are still discussed contradictorily^{[7,11,14-16]}. Especially the osteoconductive property of bone allografts leads to a significant higher volume stability compared to autologous bone, although it is still resorbable and will be degraded into autologous bone. Demineralized freeze-dried bone allografts were shown to be osteoinductive and osteoconductive due to the release of bone morphogenetic proteins^{[17]}, although clinical outcomes comparing mineralized and demineralized freeze-dried bone allografts were reported to be similar^{[18]}. Studies *in vitro* and animal investigations revealed osteoinductive functions of demineralized freeze-dried bone allografts by recruiting cells and ectopic bone formation^{[19]}. Disadvantages of allogeneic materials may be a protracted vascularization, slow remodeling and resorption or longer time for osseointegration, and the risk of immunogenic reactions^{[15-18]}.

Third, several experimental studies on animal models^{[20,21]}, clinical studies^{[22-24]}, and an earlier meta-analysis^{[25]} indicated that platelet-rich plasma (PRP) can increase new bone formation in maxillary sinus augmentation when used in combination with autologous or allogeneic graft material. The use of PRP is based on the premise that it contains large quantities of growth factors, including platelet derived growth factor, insulin-like growth factor-1, and transforming growth factor-β that may enhance osteogenesis^{[26-28]}. However, a number of recent systematic reviews and meta-analyses came to the conclusion that PRP has no significant impact on bone formation as well as on implant survival in maxillary sinus augmentation^{[29-31]}.

In this regard novel strategies are needed for improving guided bone regeneration (GBR) in oral surgery prior to implant placement, particularly in maxillary sinus augmentation (GBR-MSA) and in lateral alveolar ridge augmentation (LRA).

It appears feasible to further improve GBR-MSA by the application of stem cells (for recent reviews on the use of stem cells in regenerative dentistry^{[35-37]}). Among the different types of mesenchymal stem cells, cells derived from adipose tissue (either freshly isolated or culture-expanded) have emerged as a promising tool for GBR^{[4,38,39]}. The freshly isolated cells are named stromal vascular fraction or ADRCs, and the cultured cells are named adipose-derived stem cells (ASCs). It should be mentioned that some studies on animal models suggested that bone marrow stem cells (BMSCs) may demonstrate greater differentiation into osteoblasts than ADRCs and ASCs^{[40,41]} and might be more useful than ADRCs or ASCs in GBR-MSA^{[42]}. However, bone marrow has a significantly lower stem cell density than adipose tissue (0.01% vs 5%), and harvesting adipose tissue is much less painful than harvesting bone marrow because the former is less invasive than the latter^{[43-45]}. Furthermore, focusing exclusively on the potential to differentiate into osteoblasts would fail to take into account the known effect of indirect stimulation of bone regeneration by ADRCs and ASCs, which led to the same amount of measured regenerated bone volume after 6 wk in a study that compared the bone regeneration effect of BMSCs and ASCs in a rabbit craniectomy model^{[40]}.

Furthermore, several studies demonstrated that the combination of ADRCs or ASCs with an OIS is a more effective strategy for GBR than the use of an OIS alone (reviewed in^{[46-48]}). Moreover, it was hypothesized that the application of ADRCs or ASCs in combination with an OIS and osteogenic/angiogenic growth factors may help to optimize clinical procedures^{[46]}. In this regard the following must be kept in mind: (1) Compared to cultured and potentially modified ASCs, freshly isolated, unmodified ADRCs have the advantage of lower safety requirements because it is not necessary to culture and/or modify the cells; (2) Several non-enzymatic and enzymatic systems for isolating ADRCs were developed (reviewed in^{[49]}) and reported that cell yield may vary considerably^{[50]}. Moreover, it was shown that in general, non-enzymatic isolation of ADRCs yielded fewer cells than enzymatic (mechanical) isolation^{[51,52]}. To our knowledge, the greatest difference in cell yield between enzymatic and non-enzymatic isolation of ADRCs was reported for the Transpose RT system and the proprietary Matrase Reagent (both from InGeneron, Inc., Houston, TX, United States)^{[53]}; and (3) Some clinical studies on cell-based therapies reported the production of donor-specific antibodies after application of allogeneic cells^{[54,55]}. This is not the case when using autologous cells.

In summary, the combination of enzymatically isolated, UA-ADRCs with PRGF-2 and an OIS may be optimal for GBR-MSA.

HENK-JAN P RINS ET AL: "Bone Regeneration Using the Freshly Isolated Autologous Stromal Vascular Fraction of Adipose Tissue in Combination With Calcium Phosphate Ceramics: Bone Regeneration With Autologous Fat Stem Cells", STEM CELLS TRANSLATIONAL MEDICINE, vol. 5, no. 10, 7 July 2016 (201 6-07-07), pages 1362-1374, discloses bone regeneration in patients undergoing maxillary sinus floor elevation (MSFE) for dental implant placement using freshly isolated autologous stromal vascular fraction of adipose tissue in combination with calcium phosphate ceramics scaffolds. After isolation of the stromal vascular fraction, cells can be shortly stimulated with the growth factors before seeding the stimulated cells onto a carrier material (calcium phosphate). The difference to the present invention as defined in the claims lies in the use of PRGF-2 as per component ii) of claim 1 instead of unspecified growth factors, i.e. HENK-JAN P RINS ET AL does not refer to PRGF-2, rather the cells are at best shortly stimulated with unspecified growth factors before their contact with the calcium phosphate carrier, which is regarded as osteoconductive in the literature. A further difference lies in the claimed component "iii) osteoinductive scaffold (OIS)" as defined in claim 1, as in HENK-JAN P RINS ET AL the cells are applied to the bone substitute β-TCP or BCP, which are not OIS as they lack the osteoinductive potential.

PANAGIOTIS DRAGONAS ET AL: "Plasma rich in growth factors (PRGF) in intraoral bone grafting procedures: A systematic review", JOURNAL OF CRANIO-MAXILLO-FACIAL SURGERY, vol. 47, no. 3, 17 January 2019 (2019-01-17), pages 443-453, reviews in detail the use of combinations of plasma rich in growth factors (PRGF) in intraoral bone grafting procedures using bone scaffolds. PANAGIOTIS DRAGONAS ET AL states that outcomes of PRGF on new bone formation post extraction and on maxillary sinus augmentation when combined with other biomaterials were conflicting. Meta-analysis could not be conducted for any variables due to the heterogeneity of selected studies. Limited evidence exists on the effects of PRGF in different intraoral bone grafting procedures, with some benefit reported on soft tissue healing and post-operative symptomatology. As this platelet concentrate is commonly used in clinical practice, further research is needed to fully assess its clinical indications and effectiveness.

DEBORAH CHICHARROET AL: "Combined plasma rich in growth factors and adipose-derived mesenchymal stem cells promotes the cutaneous wound healing in rabbits", BMC VETERINARY RESEARCH, vol. 14, no. 1, 21 September2018 (2018-09-21) discloses that a combination of plasma rich in growth factors (PRGF®-Endoret® Technology) and adipose-derived mesenchymal stem cells promotes the cutaneous wound healing in rabbits.

In summary, HENK-JAN P RINS ET AL lacks the PRGF and the osteoinductive scaffold, PANAGIOTIS DRAGONAS ET AL lacks the cellular aspect and DEBORAH CHICHARROET AL lacks the osteoinductive scaffold.

### DESCRIPTION OF THE INVENTION

The invention is set out in the appended claims. The embodiments of the description which do not fall within the scope of said claims are provided for illustrative purposes only and do not form part of the present invention. The present invention is based on the finding that that GBR-MSA with a combination of unmodified autologous adipose-derived regenerative cells (UA-ADRCs), wherein said UA-ADRCs are isolated from adipose tissue comprising collagenase digestion of said tissue, followed by centrifugal density gradient separation, an autologous fraction 2 of plasma rich in growth factors (PRGF-2), and an osteoinductive scaffold (OIS) is effective, leading to a significant increase in the relative amount of newly formed bone and of dense fibrous tissue as well as less unwanted new adipose tissue formation without adverse effects. Besides this, the results presented as per the present disclosure may also be of relevance to other fields of regenerative dentistry using stem cells (reviewed in^{[115-117]}), as well as for GBR in general.

The present invention is in particular based on the surprising finding that the combination of said freshly isolated UA-ADRCs, said autologous PRGF-2, and an OIS, i.e. UA-ADRC/PRGF-2/OIS is superior to the combination of PRGF-2 and the same OIS alone (PRGF-2/OIS) in GBR-MSA/LRA.

The present invention provides a kit comprising i) UA-ADRCs, ii) PRGF-2, and iii) an OIS as defined in the claims.

The UA-ADRCs are non-cultured UA-ADRCs and are isolated from in-vivo human adipose tissue as defined in the claims, e.g. isolated from a lipoaspirate, more specifically a liposuction aspirate. In some embodiments, said UA-ADRCs may be freshly isolated UA-ADRCs or cryopreserved UA-ADRCs. In some further embodiments said UA-ADRCs may be isolated from a cryopreserved lipoaspirate or a fresh non-cryopreserved lipoaspirate immediately following its harvesting from the human tissue. Said UA-ADRCs are isolated from adipose tissue comprising collagenase digestion of said tissue, followed by centrifugal density gradient separation as defined in the claims.

Said kit can be used as a medicament. In a preferred embodiment said kit is for use in bone augmentation procedures (BAP) and/or guided bone regeneration (GBR). In some embodiments said kit is for use in a)pre-implant BAP and/or GBR, b)immediate implant placement, or a combination of both, i.e. c) pre-implant BAP and/or GBR in combination with its use in implant placement.

In a preferred embodiment the kit is for use in oral surgery.

In a preferred embodiment said use is for pre-implant bone augmentation selected from the group consisting of lateral and horizontal alveolar ridge augmentation, socket preservation, bone grafting following cystectomies and rehabilitation of the edentulous posterior maxilla. In further preferred embodiment said use is for rehabilitation of the edentulous posterior maxilla. Furthermore, the use may comprise a bone preparation for implant placement, for example maxillary sinus augmentation (MSA) and/or lateral ridge augmentation (LRA) prior to implant placement.

The invention further relates to the use of UA-ADRCs in a method of treatment of a human subject as outlined for the kit above.

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

As used herein, the term "non-cultured" and "isolated from in-vivo human adipose tissue" means isolated from a human fat tissue without subsequent culturing or in-vitro expansion. The adipose tissue may be brown or white adipose tissue, derived from subcutaneous, omental/visceral, mammary, gonadal, or other adipose tissue site. Preferably, the adipose tissue is subcutaneous white adipose tissue. A convenient source of adipose tissue is from liposuction surgery thereby providing a "lipoaspirate" "liposuction aspirate" as a source of UA-ADRCs, however, the source of adipose tissue or the method of isolation of adipose tissue is not critical to the invention. If UA-ADRCs are desired for the intended use in the methods of the present invention, the adipose tissue will be isolated from a human subject.

As used herein, the term "ADRCs" refers to a heterogeneous or mixed population of cells found in adipose tissue by collagenase digestion. This population includes adult stem cells, endothelial progenitor cells, endothelial cells, vascular smooth muscle cells, and leukocytes. "UA-ADRCs" or "freshly isolated UA-ADRCs" refer to unmodified autologous isolated cells from adipose tissue which has not been expanded (i.e. not cultured in-vitro), wherein said UA-ADRCs can be used freshly or can be cryopreserved for later use. Similarly, the lipoaspirate or liposuction aspirate can be treated immediately after its isolation from the human subject for harvesting of the UA-ADRCs or can be cryopreserved for later harvesting of said UA-ADRCs. Extraction and concentration of ADRCs for clinical use without requiring a culturing step is known from the prior art (see e.g. Lin K, Matsubara Y, Masuda Y, Togashi K, Ohno T, Tamura T, Toyoshima Y, Sugimachi K, Toyoda M, Marc H, Douglas A, Cytotherapy. 2008; 10(4):417-26) or as outlined in Example 2 of the below experimental section. As used herein, the term "PRGF-2" refers to fraction 2 obtained from centrifuged blood and activated with the PRGF activator according to the procedure well known to a skilled person in the art, e.g. via using the plasma transfer device PTD2 along the lines of the endoret® protocol (http://bti-biotechnologyinstitute.com/web/uploads/media/client/0001/04/2015_us_protocolo_endoret_stepbystep_aparato_locomotor.pdf).

As used herein, the term "osteoinductive scaffold (OIS)" refers to a scaffold stimulating the osteoprogenitor cells to differentiate into osteoblasts that begin new bone formation, thereby not only serving as a scaffold for currently existing osteoblasts, but also triggering the formation of new osteoblasts.

As used herein, the term "bone augmentation procedures (BAP)" refers to a procedure of growing a bone typically needed when the current bone mass is not suitable to successfully incorporate an implant, e.g. a dental implant to be incorporated into a jaw. In other words, when bone mass is too thin or soft or the bone volume is not sufficient to keep an implant in place, said bone augmentation helps the bone to primarily stabilize the dental implant and keep it secure until osseointegration of the implant occurs.

As used herein, the term "guided bone regeneration (GBR)" refers to ridge augmentation of bone regenerative procedures, in particular the development of hard tissue growth on an alveolar ridge to allow stable placement of dental implants, including lateral and horizontal alveolar ridge augmentation.

### DESCRIPTION OF DRAWINGS

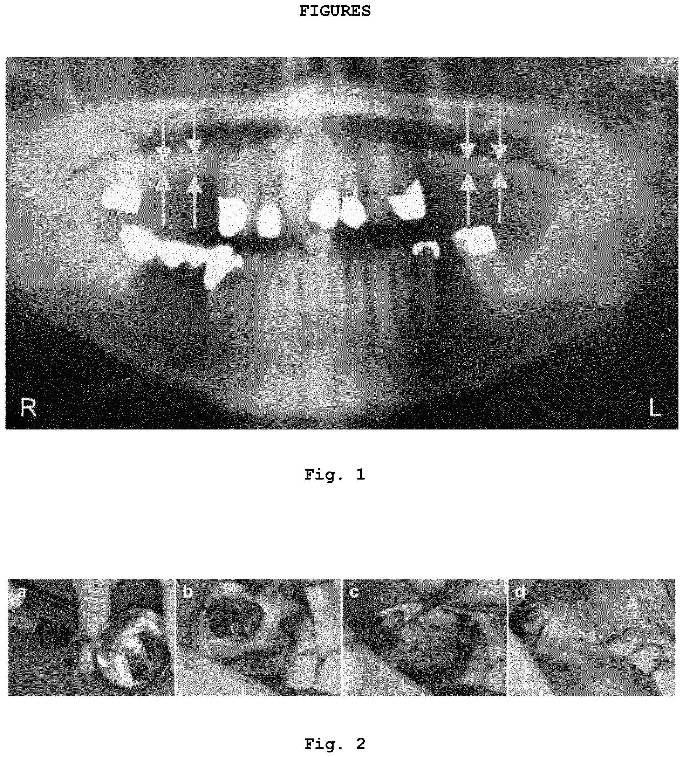

**Figure 1** is a panoramic radiograph at T0. The arrows indicate the reduced bone height of the edentulous posterior maxilla below the antrum and the ridge crest of less than 3 mm on both sides. R: Right; L: Left.
**Figure 2** provides details of the procedures performed immediately before and during the first surgery (guided bone regeneration-maxillary sinus augmentation). A: Rehydration of the MCBPA with PRGF-2 and UA-ADRCs; B: Preparation of the right maxillary sinus with a lateral external window after atraumatic extraction of tooth # 14 (the Schneiderian membrane was elevated); C: Filling of the right sinus cavity with loosely packed MCBPA/PRGF-2/UA-ADRCs; D: Achievement of primary closure without tension on the right side using horizontal mattress as well as a continuous half-hitch sutures. MCBPA: Mineralized cancellous bone particulate allograft; PRGF-2: Fraction 2 of plasma rich in growth factors; UA-ADRCs: Unmodified autologous adipose-derived regenerative cells.

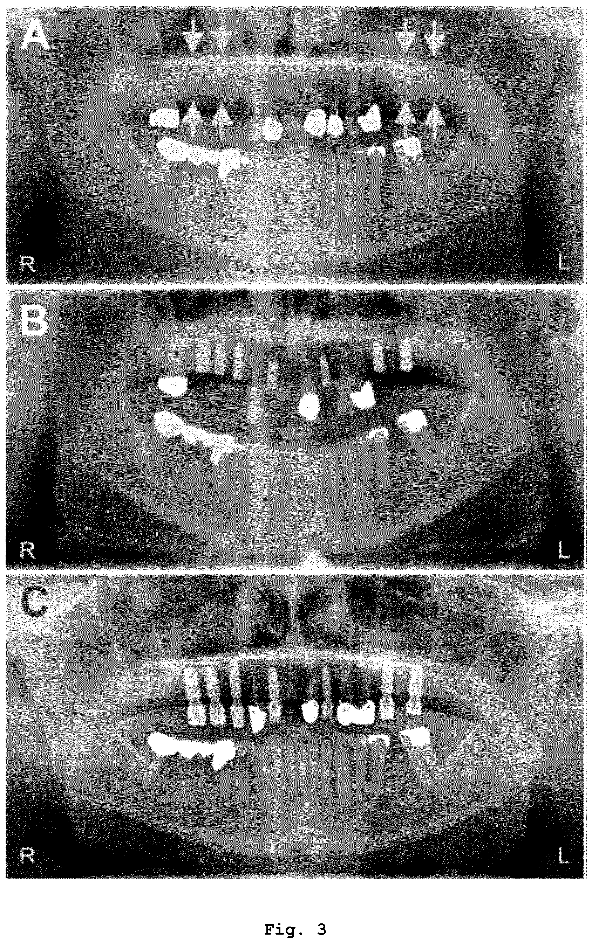

**Figure 3** shows the clinical findings. A: Panoramic radiograph taken immediately after the first surgery (GBR-MSA). The arrows indicate the restored bone height of the edentulous posterior maxilla on both sides; B: Panoramic radiograph taken after the third surgery (placement of implants at 34 wk after GBR-MSA); C: Panoramic radiograph taken at 32 mo after the first treatment. R: Right; L: Left; GBR-MSA: Guided bone regeneration maxillary sinus augmentation.

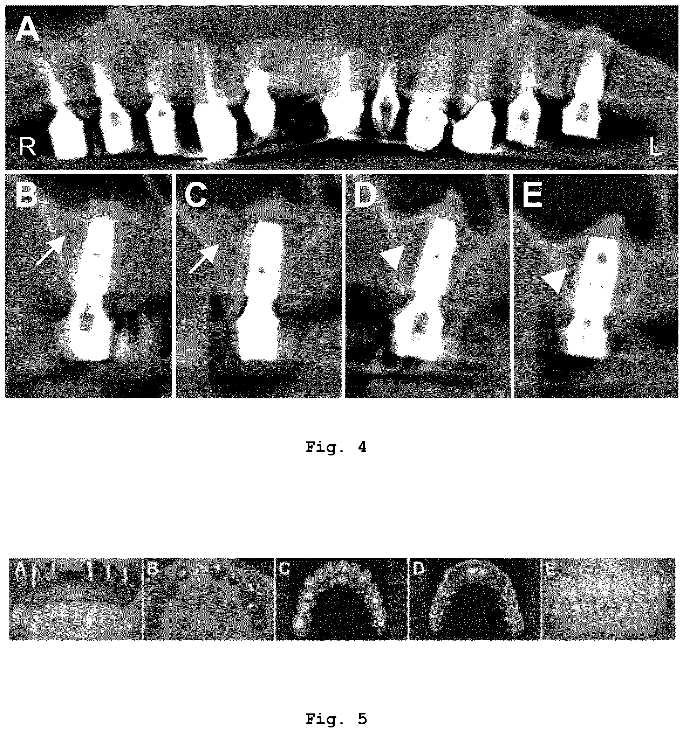

**Figure 4** provides digital volume tomography radiographs taken after the fourth surgery (1 year after the placement of implants and 20 mo after guided bone regeneration-maxillary sinus augmentation). A: Panoramic view; B-E: Detailed view on selected regions 15 (B), 16 (C), 25 (D), and 26 (E). With the application of UA-ADRCs the bone around the implants in regions 15 and 16 appeared larger in area and denser (arrows in B, C) than without the application of UA-ADRCs in regions 25 and 26 (arrowheads in D, E). UA-ADRCs: Unmodified autologous adipose-derived regenerative cells.
**Figure 5** provides documentation of clinical outcome. A, B: Intraoral ventral (A) and occlusal (B) views on the healing abutments in regions 12, 14, 15, 16, 22, 25, and 26 after the fourth treatment (1 year after the placement of implants and 20 mo after GBR-MSA). Note that the teeth # 13, 21, 23, and 24 are crowned; C, D: External (C) and internal (D) view of the prosthetic telescopic bridge; E: Intraoral view of the final prosthetic reconstruction. GBR-MSA: Guided bone regeneration maxillary sinus augmentation.

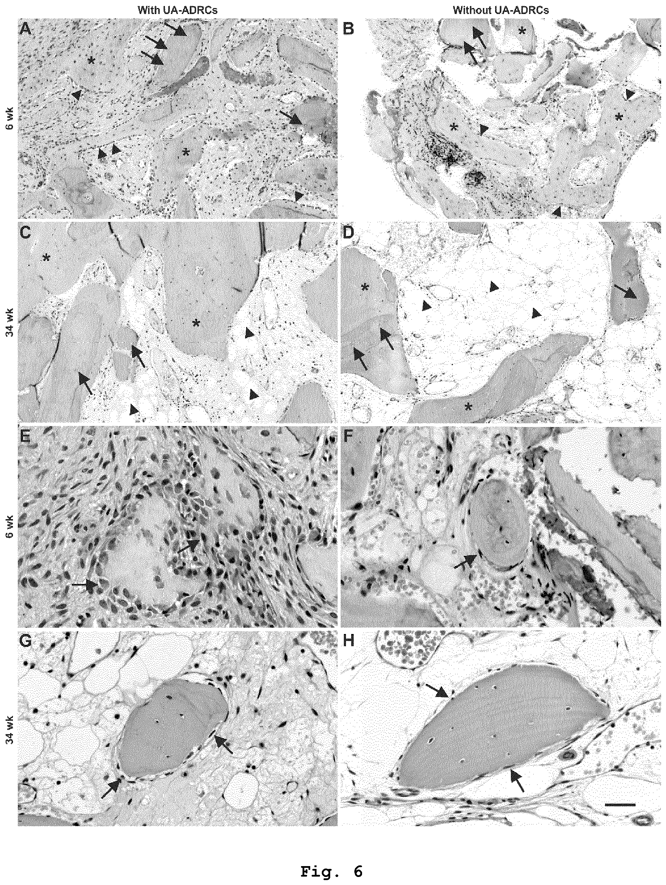

**Figure 6** provides histological findings. Representative photomicrographs of 3 µm thick paraffin sections stained with hematoxylin and eosin s of biopsies that were collected at 6 wk (A, B, E, F) or 34 wk (C, D, G, H) after GBR-MSA with the application of UA-ADRCs (A, C, E, G) or without UA-ADRCs (B, D, F, H). In A-D the asterisks indicate newly formed bone and the arrows indicate empty osteocyte lacunae in allogeneic fragments. Furthermore, in A and B the arrowheads point to osteoblasts with underlying osteoid, while in C and D the arrowheads indicate adipocytes. In E-H the arrows indicate cells on the surface of newly formed bone. The scale bar in H represents 150 µm in A-D and 75 µm in E-H. GBR-MSA: Guided bone regeneration maxillary sinus augmentation; UA-ADRCs: Unmodified autologous adipose-derived regenerative cells.

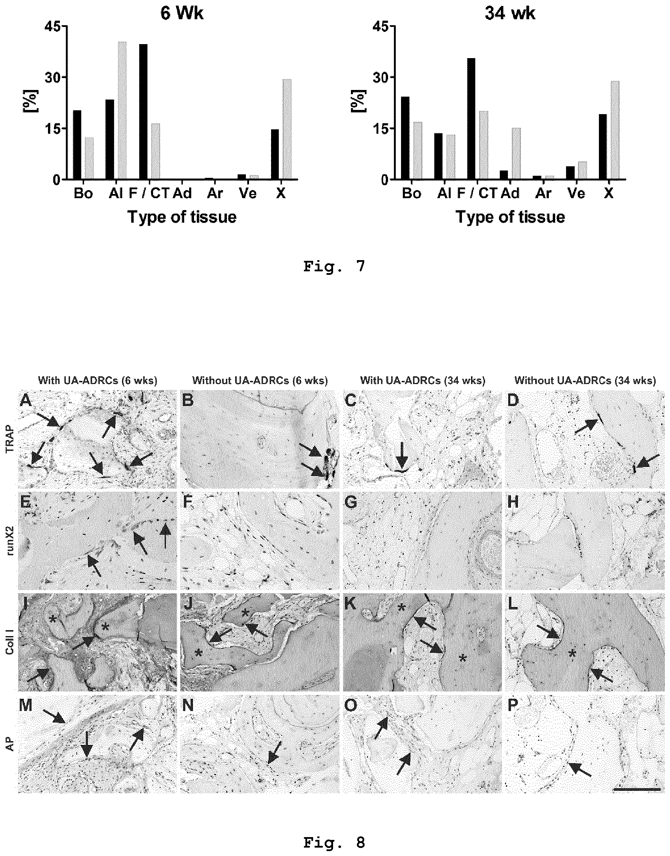

**Figure 7** provides results of histomorphometric analyses. Relative amounts (area/area) of bone (Bo), allograft (Al), fibrin and connective tissue (F/CT), adipocytes (Ad), arteries (Ar), veins (Ve), and artifacts (X) in 3 µm thick paraffin sections stained with hematoxylin and eosin of biopsies that were collected at 6 wk (on the left) or 34 wk (on the right) after GBR-MSA with the application of UA-ADRCs (closed bars) or without UA-ADRCs (gray bars). GBR-MSA: Guided bone regeneration maxillary sinus augmentation; UA-ADRCs: Unmodified autologous adipose-derived regenerative cells.
**Figure 8** provides histological findings. Representative photomicrographs of histochemical detection of tartrate-resistant acid phosphatase (TRAP) (A-D) as well as of immunohistochemical detection of Runt-related transcription factor 2 (runX2) (E-H), collagen 1 (Coll 1) (I-L), and alkaline phosphatase (AP) (M-P) in 3 µm thick paraffin sections of biopsies that were collected at 6 wk (A, B, E, F, I, J, M, N) or 34 wk (C, D, G, H, K, L, O, P) after GBR-MSA with the application of UA-ADRCs (A, C, E, G, I, K, M, O) or without UA-ADRCs (B, D, F, H, J, L, N, P). In A-D the arrows point to osteoclasts, in E to osteoblasts, in I-L to type I collagen in osteoid seams, and in M-P to AP immunostaining found in osteoblasts, osteoblast-like cells, and fibroblasts in the intertrabecular connective tissue. Furthermore, in I-L the asterisks indicate type I collagen in the matrix of newly formed bone trabeculae. The scale bar in P represents 200 µm in A-D and I-P, and 100 µm in E-H. GBR-MSA: Guided bone regeneration maxillary sinus augmentation.

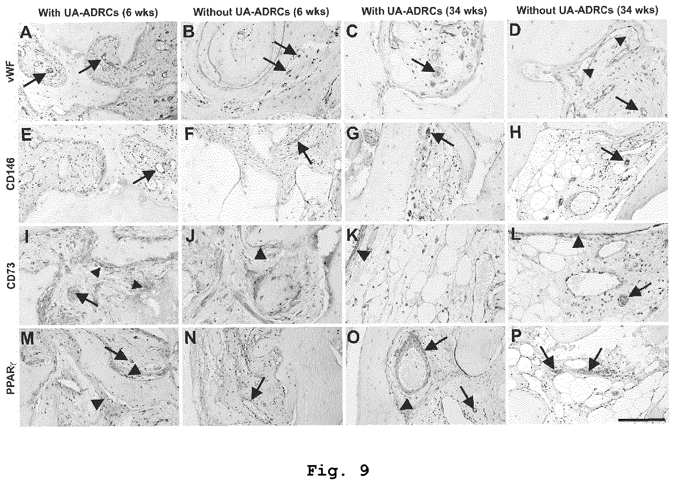

**Figure 9** provides histological findings. Representative photomicrographs of immunohistochemical detection of factor VIII/von Willebrand factor (vWF) (A-D), CD146 (E-H), CD73 (I-L), and peroxisome proliferator-activated receptor gamma (PPARγ) (M-P) in 3 µm thick paraffin sections of biopsies that were collected at 6 wk (A, B, E, F, I, J, M, N) or 34 wk (C, D, G, H, K, L, O, P) after GBR-MSA with the application of UA-ADRCs (A, C, E, G, I, K, M, O) or without UA-ADRCs (B, D, F, H, J, L, N, P). In all panels the arrows point to vessels. Furthermore, the arrowheads indicate sinusoidal vessels in D, and fibroblasts and osteoblasts in I-L, M, and O. The scale bar in P represents 200 µm in A-D and I-P, and 100 µm in E-H. GBR-MSA: Guided bone regeneration maxillary sinus augmentation; UA-ADRCs: Unmodified autologous adipose-derived regenerative cells.

### EXAMPLES

### AIM, CASE SUMMARY AND CONCLUSION OF THE SUBSEQUENT EXAMPLES

The aim of the present study as outlined in the present examples section was to test (using a first-in-human, split-mouth single case study design) the hypothesis that in GBR-MSA the combination of freshly isolated, unmodified autologous adipose derived regenerative cells (UA-ADRCs), fraction 2 of plasma rich in growth factors (PRGF-2), and an osteoinductive scaffold (OIS) is superior to the combination of PRGF-2 and the same OIS alone. Due to the fact that preliminary data were not available, the present study tested the null hypothesis that the combination of UA-ADRCs, PRGF-2, and an OIS in GBR-MSA is not more effective than the combination of PRGF-2 and the same OIS alone.

A 79-year-old patient was treated with a bilateral external sinus lift procedure as well as a bilateral lateral alveolar ridge augmentation. GBR-MSA/LRA was performed with UA-ADRC/PRGF-2/OIS on the right side, and with PRGF-2/OIS on the left side. Biopsies were collected at 6 wk and 34 wk after GBR-MSA/LRA. At the latter time point implants were placed. Radiographs (32 mo follow-up time) demonstrated excellent bone healing. No radiological or histological signs of inflammation were observed. Detailed histologic, histomorphometric, and immunohistochemical analysis of the biopsies evidenced that UA-ADRC/PRGF-2/OIS resulted in better and faster bone regeneration than PRGF-2/OIS. In other words, it has been demonstrated that the combination of freshly isolated, unmodified autologous adipose-derived regenerative cells, fraction 2 of plasma rich in growth factors and an osteoinductive scaffold is superior to the combination of fraction 2 of plasma rich in growth factors and the same osteoinductive scaffold alone. This novel procedure may contribute to a decreased healing period and increased bone quality in rehabilitation of the edentulous posterior maxilla as well as in other regenerative techniques in pre-implant bone augmentation procedures. In conclusion, GBR-MSA with UA-ADRCs, PRGF-2, and an OIS shows effectiveness without adverse effects.

### EXAMPLE 1 - CASE PRESENTATION

### Chief complaints

A 79-year-old male patient presented with a partly failing maxillary dentition to the clinic of the principal investigator who specialized in periodontology and implant dentistry. The patient reported that his major concern was a functional occlusion resulting in restoration of an aesthetic smile.

### History of present illness

The patient reported extensive restorative treatment in the past as well as the loss of several premolar and molar teeth.

### History of past illness

No specific past illness was reported that was directly related to the present illness. However, the patient reported reduced oral hygiene in the past, including lack of supragingival plaque control and limited motivation for oral hygiene.

### Personal and family history

No specific personal and family history was reported that was directly related to the present illness.

### Physical examination upon admission

The clinical examination upon admission revealed a reduced vertical dimension of occlusion and loss of several premolar and molar teeth. Furthermore, advanced periodontal defects were present around several teeth in the anterior maxilla as well as around maxillary and mandibular molar teeth. Most of the remaining maxillary teeth had a guarded to hopeless prognosis.

### Laboratory examinations

No laboratory examinations were performed upon admission.

### Imaging examinations

A panoramic radiograph was performed upon admission (i.e., at time point T0; the time course of the present study is summarized in below Table 1) showed the loss of several premolar and molar teeth and revealed that the residual bone height of the edentulous posterior maxilla below the antrum and the ridge crest was less than 3 mm on both sides (Class D according to^{[32]}) (Figure 1).

**Table 1 Overview on the treatments performed in the present study.**

| **Treatments** | **Time point** | **Procedures** |
|---|---|---|
| Pre-phase | T0 | Evaluation of the patient |
| Preparatory steps | T1 (4 mo after T0) | Preparation of plasma rich in growth factors (PRGF-2) |
| | | Isolation of unmodified, autologous adipose derived regenerative cells (UA-ADRCs) |
| 1 | T1 (GBR-MSA) | Extraction of tooth # 14 |
| | | Ridge preservation and external sinus lift procedures |
| 2 | T2 (6 wk after T1) | Extraction of teeth # 11, #12 and #22 |
| | | Collection of the first biopsies |
| 3 | T3 (34 wk after T1) | Placement of implants |
| | | Collection of the second biopsies |
| 4 | T4 (1 yr after T3) | Extraction of tooth #18 |
| | | Placement of healing abutments |
| 5 | T5 (6 wk after T4) | Placement of the definitive prosthetic telescopic bridge |
| | T6 (32 mo after T1) | Last radiologic follow-up |

| | | |
|---|---|---|
| In order to prevent potential identification of the patient the timeline was coded. | | |

The residual bone height of the edentulous posterior maxilla as well as of the alveolar bone crest height around the remaining maxillary teeth did not justify immediate implant placement. Therefore, extensive bone augmentation procedures (GBR-MSA) were necessary prior to implant placement. According to the literature, residual bone height of the edentulous posterior maxilla below the antrum and the ridge crest of less than 3 mm shall be treated with a lateral approach involving a bone grafting material and delayed implant placement^{[33]}. To date, the combination of PRGF-2 instead of PRP and an OIS appears to be the most advanced procedure for GBR-MSA. This is due to the fact that a recent experimental study on rats showed that PRGF-2 has more availability for bone regeneration than PRP^{[34]}. The use of a combination of PRGF-2 (prepared using the PRGF-Endoret technology; BTI, Miñano, Spain) and a mineralized cancellous bone particulate allograft (MCBPA) (Puros Cancellous Particulate Allograft; Zimmer Biomet Dental, Palm Beach Gardens, FL, United States) is the current standard procedure for GBR-MSA at the Clinic for Periodontology and Implantology (Hamburg, Germany).

Taking the diagnosis of the patient into account, there is a justification for treatment with a novel combination of UA-ADRCs, PRGF-2, and an OIS in a first-in-human, split-mouth single case study, carefully controlled by comprehensive histologic, design-based stereologic, and immunohistochemical analysis of biopsies of the newly formed bone collected at different time points after GBR-MSA.

### EXAMPLE 2 - TREATMENT

The present single-case study was approved by the ethics committee of the Federal Dental Association Hamburg (Hamburg, Germany) (no. PV5211). Written informed consent was obtained from the patient to participate in this study after verbal and written information provided by the principal investigator. During a 4 mo pre-phase the patient's reduced oral hygiene was significantly improved by oral hygiene advice and supragingival plaque control.

During the first treatment, venous blood (8 × 9 mL = 72 mL) was withdrawn from the patient's arm and was processed into PRGF using the PRGF-Endoret technology (BTI, Miñano, Spain) according to the manufacturer's instructions for use. After centrifugation, PRGF-2 was collected and activated with PRGF Activator (BTI) .

Furthermore, human subcutaneous adipose tissue was obtained using liposuction. To this end, the periumbilical abdominal area was surgically disinfected. Then, local anesthesia was achieved by infiltrating the periumbilical subcutaneous adipose tissue with 150 mL of modified Klein tumescent solution^{[57]}, comprising lactated Ringer solution, adrenaline (1:1000; 1 mg/mL) and 2% lidocaine (20 mg/mL). Fifteen minutes later a stab incision was made 15 cm lateral of the umbilicus, bilaterally. Lipoaspiration was performed using the Coleman method^{[58]} using a 4-hole blunt tipped cannula (3 mm × 150 mm) (part of the LCK-15 Lipoaspiration Collection Kit; InGeneron) and a 60 cm³ Luer-Lock Toomey-Syringe (also part of the LCK-15 Kit; InGeneron). After liposuction, pressure was applied to the wounds. Then the wounds were closed using adhesive bandage strips (Leukosilk; BSN Medical GmbH, Hamburg, Germany). The lipoaspirate (100 mL) was divided into four aliquots of 25 mL each. Then, all aliquots were processed using the Transpose RT system (InGeneron) for isolating UA-ADRCs from the adipose tissue. Specifically, each aliquot was incubated together with Matrase Reagent (InGeneron) for 1 h. The latter was performed in the processing unit under agitation at 39 °C according to the manufacturer's instructions for use. The total procedure time was 70 min.

Approximately 7 g (2 × 2 cubic centimeters of 0.25-1.0 mm particle size and 1 x 3 cubic centimeters of 1-2 mm particle size) of Puros Cancellous Particulate Allograft (Zimmer Biomet Dental, Palm Beach Gardens, FL, United States) were rehydrated with 1.5 mL of PRGF-2 and a suspension of UA-ADRCs (approximately 50 × 10⁶ cells in 3 mL saline) (hereafter referred to as MCBPA/PRGF-2/UA-ADRCs). This was done within 30 min after completion of the PRGF processing and immediately after completion of isolating the UA-ADRCs (Figure 2A). Another 7 g of the same MCBPA were rehydrated with 1.5 ml of PRGF-2 and 3 mL of saline (hereafter referred to as MCBPA/PRGF-2/saline).

After the patient was prepared for surgery and anesthetized *via* local infiltration with Ultracain-DS Forte (Sanofi-Aventis, Frankfurt/Main, Germany), the maxillary tooth 14 was extracted using a minimal traumatic approach with periotomes. Following careful extraction with special emphasis on preservation of the buccal plate of bone, the extraction sockets were examined for potential perforation and fenestration. Curettage of the extraction site was performed to remove all soft tissue debris as well as granulation tissue and to promote healing by stimulating bleeding from the osseous base. Additionally, a bilateral external sinus lift procedure using the Tatum technique^{[59]} was performed. The lateral window access was prepared in the areas of the first molars on both sides using a very atraumatic piezosurgery approach (Mectron, Cologne, Germany) to a size of approximately 10 mm × 7 mm (length × height) in order to allow for sufficient overview into the sinus cavity and to minimize the reduction of cortical bone for overall stability (Figure 2B). Then, the MCBPA/PRGF-2/UA-ADRCs was loosely packed into the prepared right maxillary sinus (Figure 2C), and the MCBPA/PRGF-2/saline into the prepared left maxillary sinus. Afterwards, all sites were covered with a resorbable native pericardium membrane (CopiOs Extend Membrane; Zimmer Biomet Dental) in order to promote the ingrowth of new bone by excluding epithelial migration into the grafted sites. Finally, a soft tissue flap extension^{[60,61]} was surgically performed and primary closure without tension was achieved using vertical and horizontal cross mattress sutures using Gore-Tex Suture (W.L. Gore and Associates, Inc., Flagstaff, AZ, United States) (Figure 2D). The anterior maxillary teeth 11, 12 and 22 were initially preserved for aesthetic reasons and scheduled for extraction 6.5 wk later (Figure 3A).

Analgesics (Paracetamol 500 mg, t.i.d.) and prophylactic antibiotics (Amoxicillin 500 mg, t.i.d.) were prescribed for 7 d postoperatively. Tooth brushing in the surgical area was restricted for the first 2 wk. In addition, chlorhexidine mouthwash was prescribed to maintain the oral flora and prevent infection. Sutures were removed on the 10^{th} postoperative day, and routine monitoring appointments were held at monthly intervals to evaluate healing.

Six weeks after the first treatment, the patient was reappointed and the maxillary anterior teeth # 11, 12, and 22 were extracted (second treatment) using the same approach as applied to the maxillary posterior tooth # 14 during the first treatment. During this procedure, biopsies of the areas grafted during the first treatment with the MCBPA/PRGF-2/UA-ADRCs (right) and the MCBPA/PRGF-2/saline (left) were collected using a trephine bur (length: 18 mm; inner diameter: 2.6 mm; outer diameter: 3.2 mm; Trepan Bur 227A.204.032; Komet Dental, Lemgo, Germany). Biopsies were fixed by immersion in 4% buffered formaldehyde at room temperature. After at least 1 d of fixation they were prepared for histologic and immunohistological analysis (described in detail below).

The postoperative management (prescription of analgesics, antibiotics, and chlorhexidine mouthwash, restriction of tooth brushing in the surgical area, removal of sutures, and routine monitoring) was the same as performed after the first treatment.

The patient was reappointed again at 34 wk after the second treatment and prepared for dental implant placement surgery (third treatment). Anesthesia was induced via local infiltration of Ultracain-DS Forte (Sanofi-Aventis). Then, an osteotomy for implant placement was initially prepared in the alveolar bone using a trephine bur that was identical to the one used during the second treatment (Trepan Bur 227A.204.032; Komet Dental), and biopsies of the areas grafted during the first treatment with the MCBPA/PRGF-2/UA-ADRCs (right side) and the MCBPA/PRGF-2/saline (left side) were collected (these biopsies were handled and processed in the same way as the biopsies that were collected during the second treatment). The osteotomies for implant placement were prepared by sequential cutting to the radiographically determined length with surgical drills in graduated diameters according to the dental implant manufacturer's instructions of use. Implants were then placed according to the manufacturer's recommendations (ASTRA TECH implants with cover screw, Dentsply, Mannheim, Germany). Implants of the following dimensions were inserted into the finally prepared osteotomies: regions 16 and 26: 5.0 S × 11 mm; region 14: 4.0 S × 13 mm; regions 12, 15, and 25: 4.0 S × 11 mm; and region 22: 3.5 S × 11 mm. All implants achieved a high primary stability of 25-30 Ncm insertion torque. The implant access holes were closed with cover screws prior to primary soft tissue closure as performed during the first treatment (Figure 3B). The postoperative management was the same as performed after the first and second treatments.

Twelve months after the third treatment, radiographs were taken and demonstrated excellent bone healing around the dental implants, within the augmented sinus, and the extraction sockets (Figure 4) (fourth treatment) (note that the long time between the third and the fourth treatment was due to constraints of time on the side of the patient; from a medical point of view the fourth treatment could have been performed already at 4 mo after the third treatment). No radiological signs of inflammation were observed. The patient was reappointed again and prepared for uncovering of the implants and implant abutment placement surgery as well as extraction of tooth #18. Anesthesia was induced via local infiltration of Ultracain-DS Forte (Sanofi-Aventis). Localized mucoperiosteal flaps were raised and the cover screws were removed from the implant access holes. The implant access holes were rinsed using 2% chlorhexidine solution and were then covered with ASTRA Tech Implant healing abutments ('Gingivaformer') of the following dimensions: regions 16 and 26: 5.5 mm × 4.0 mm; regions 12, 14, 15, and 25: 4.5 mm × 4.0 mm; and region 22: 3.5 mm × 4.0 mm.

Prior to closure of the implant access hole, the healing abutment screws were covered with 2% chlorhexidine gel in order to minimize bacterial contamination. The healing abutments were placed using a torque of 20 Ncm according to the manufacturer's recommendations. All implants were surrounded with very sufficient three-dimensional bone volume and demonstrated excellent stability (Figure 4). No clinical signs of inflammation were observed. Tooth # 18 was carefully extracted using a piezosurgery device as described above. Following achievement of primary closure around the healing abutments as described for the other treatments, the same postoperative care regimen was administered as described above and the sutures were removed approximately 10 d postoperatively. Six weeks after placement of the healing abutments, the definitive prosthetic telescopic bridge (prepared by Dr. Johanna Hevelke, Winsen/Luhe, Germany) was placed.

Histologic processing of the biopsies was performed at the Department of Orthodontics, Center of Dento-Maxillo-Facial Medicine, University of Bonn, Germany. To this end, the fixed biopsies were decalcified in 4.1% EDTA solution at room temperature for about 7 d, changing the EDTA solution every 24 h. Then, the biopsies were hydrated, followed by rehydration in an ascending series of ethanol. Afterwards they were embedded in paraffin and cut into 3 µm thick serial longitudinal sections. The sections were mounted on Superfrost Plus slides (Gerhard Menzel, Braunschweig, Germany). Sections representing positions within the biopsies near the longitudinal axis were stained with hematoxylin and eosin stain, or were processed by immunohistochemistry. Finally, all sections were coverslipped with DePeX (Serva, Heidelberg, Germany). Relative amounts (area/area) of bone, allograft, connective tissue and fibrin, adipocytes, arteries, and veins were determined on the sections that were stained with hematoxylin and eosin stain using point counting as described in detail in^{[56]} (Figure 1B). The distance between the points was 110 µm in XY directions, resulting in a mean total number of 943 (range, 662-1172) points per section. Analyses were performed with a computerized stereology workstation consisting of a modified light microscope (Axioskop; Carl Zeiss Microscopy, Jena, Germany) with Plan-Neofluar objectives 2.5 × (numerical aperture [NA] = 0.075) and 10 × (NA = 0.3) (Carl Zeiss Microscopy), motorized specimen stage (BioPrecision2; Ludl Electronics, Hawthorne, NY, United States), stage controller (MAC 6000 XY; Ludl Electronics), focus encoder (MT 1271; Heidenhain, Traunreut, Germany), CCD color video camera (1,600 x 1,200 pixels; MBF Bioscience, Williston, VT, United States), and stereology software (Stereo Investigator version 10; MBF Bioscience).

After deparaffinizing and rehydrating, sections were rinsed for 10 min in TBS. Histochemical detection of tartrate-resistant acid phosphatase was performed with a specific Acid Phosphatase staining kit (Sigma-Aldrich, Steinheim, Germany) according to the staining protocol of the manufacturer. For immunohistochemistry, endogenous peroxidase was blocked in a methanol/H₂O₂ (Merck, Darmstadt, Germany) solution for 45 min in the dark. Then, sections were pretreated with TBS containing 1% bovine serum albumin at room temperature for 20 min. Afterwards, sections were digested with 0.4% pepsin at 37 °C for 10 min, followed by incubation with the primary antibodies in a humid chamber. Table 2 below summarizes details of the antibodies and the incubation protocols.

**Table 2 Details of antibodies and incubation protocols used in the present study**

| **Antibody** | **Isotype** | **Manufacturer** | **Dilution/incubation** |
|---|---|---|---|
| runX2 | Goat polyclonal | Santa Cruz Biotechnology (Dallas, TX, United States) | 1:30, 4 °C, ON |
| Collagen Type I | Rabbit monoclonal | Abcam (Cambridge, United Kingdom) | 1:400, RT, 1 h |
| Alkaline phosphatase | Rabbit polyclonal | Quartett (Berlin, Germany) | Ready to use, 4 °C, ON |
| Von Willebrand Factor | Rabbit polyclonal | Linaris (Dossenheim, Germany) | 1:200, RT, 1h |
| CD146 | Rabbit monoclonal | Abcam | 1:50, RT, 1h |
| CD73 | Mouse monoclonal | Antibodies-online (Atlanta, GA, United States | 1:100, 4 °C, ON |
| PPARγ | Mouse monoclonal | Santa Cruz Biotechnology | 1:25, 4 °C, ON |

| | | | |
|---|---|---|---|
| ON: Overnight; RT: Room temperature. | | | |

Antibody binding was detected with the peroxidase-conjugated EnVision antimouse system (Dako, Glostrup, Denmark) or the horseradish peroxidase-conjugated EnVision anti-rabbit/anti-goat secondary antibodies (Dako) that were diluted 1:50 and incubated at room temperature for 30 min. Visualization of peroxidase activity was performed using diaminobenzidine resulting in a brown staining product. Mayer's hematoxylin was used for counterstaining the sections. In order to perform specificity controls, primary antibodies were omitted and TBS or normal horse serum was applied. In other control experiments, both primary and secondary antibodies were omitted. Fetal human bone or mandibular bone (*i.e*., tissues carrying known antigens) were used as positive controls. Qualitative histological evaluations were performed blinded by one of the authors.

Digital photography was used to produce the photomicrographs shown in Figures 6 and 7. To this end, on average 41 (range, 30-45) images were captured for each composite using a computerized stereology workstation. The latter consisted of a modified light microscope (Axio Imager 2; Carl Zeiss Microscopy) with an EC Plan Neofluar 10 × objective (NA = 0.3) (Carl Zeiss Microscopy), motorized specimen stage for automatic sampling (H101A; Prior Scientific Instruments, Cambridge, United Kingdom), focus encoder (MT 1271; Heidenhain), CCD color video camera (1388 x 1040 pixels; AxioCam MRc; Carl Zeiss Microscopy), and stereology software (Stereo Investigator version 10; MBF Bioscience). The Virtual Slice module of the Stereo Investigator software (MBF Bioscience) was used to create the montages. The photomicrographs shown in Figures 8, 10, and 11 were produced by digital photography (all components from Carl Zeiss Microscopy) using an AxioCam MRc camera attached to an AxioScope 2 microscope and AxioVision 4.7 software using the following objectives: Epiplan 20 × (NA = 0.40) and Plan-Neofluar 40 × (NA = 0.75). Corel Photo-Paint X7 and Corel Draw X7 (both versions 17.5.0.907; Corel, Ottawa, Canada) were used to construct the final figures. Contrast and brightness were only marginally adjusted, which did not alter the appearance of the original materials.

### EXAMPLE 3 - OUTCOME AND FOLLOW-UP

The patient was very satisfied with the maxillary restoration regarding aesthetics, function, phonetics, and cleansibility (Figure 5). The last radiologic follow-up examination took place at 32 mo after the GBR-MSA surgery (Figure 3C).

The biopsies that were collected at 6 wk after GBR-MSA showed the formation of a network of cancellous bony trabeculae by appositional membranaceous osteogenesis in different developmental stages around allogeneic fragments. The newly formed bone (asterisks in Figure 6A and 6B) consisted of fibrous bone. Typically, newly formed bone spicules contained nuclei of allogeneic remnants that showed basophilic staining and contained empty osteocyte lacunae (arrows in Figure 6A and 6B). Most surfaces of the newly formed trabeculae were covered by osteoblasts with underlying osteoid (arrowheads in Figure 6A and 6B). With the application of UA-ADRCs a higher bone lining cell density was achieved than without UA-ADRCs. No signs of active inflammation or necrosis could be recognized in either biopsy.

The biopsies that were collected at 34 wk after GBR-MSA showed a similar morphology as the biopsies that were collected at 6 wk after GBR-MSA. However, bone formation seemed to be increased with a decreased percentage of allogeneic remnants (arrows in Figure 6C and 6D) and fibrous bone. Newly formed cancellous bone was in an advanced stage of remodeling, appearing as lamellar bone (asterisks in Figure 6C and 6D) with fibrous bone remnants incorporated. Fewer adipocytes developed after the application of UA-ADRCs than without UA-ADRCs (arrowheads in Figure 6C and 6D).

At higher magnification, the biopsy that was collected at 6 wk after GBR-MSA with the application of UA-ADRCs showed regions with early osteogenic condensations within a highly cellular surrounding. Specifically, osteoclasts appeared on the surface of natural and allogeneic bone (arrowheads in Figure 6E). Such regions with early osteogenic condensations were not observed at 6 wk after GBR-MSA without application of UA-ADRCs. Rather, osteogenesis appeared more discrete in this case (Figure 6F). Likewise, at 34 wk after GBR-MSA a higher cell density around newly formed cancellous bone was found after the application of UA-ADRCs (arrowheads in Figure 6G) than without UA-ADRCs (arrowheads in Figure 6H).

The results of the histomorphometric analysis are summarized in Figure 7. The application of UA-ADRCs resulted in a higher relative amount (area/area) of newly formed bone (+ 63% at 6 wk; + 44% at 34 wk), a higher relative amount of fibrin and collagen (+ 144% at 6 wk; + 78% at 34 wk) and a lower relative amount of adipocytes (- 83% at 34 wk) compared to the situation without application of UA-ADRCs. Besides this, the ratio of the relative amounts (area/area) of veins to arteries was 3.5 after the application of ADRCs and 4.7 without ADRCs at 34 wk after GBR-MSA.

Tartrate-resistant acid phosphatase immunostaining revealed middle-sized osteoclasts on the surface of newly formed and allogeneic bone and on debris accumulations at 6 wk after GBR-MSA, with a higher osteoclast density after the application of UA-ADRCs than without UA-ADRCs (arrows in Figure 8A and 8B). Only a few osteoclasts were found at 34 wk after GBR-MSA (arrows in Figure 8C and 8D).

Anti-runt-related transcription factor 2 immunoreactivity was found at 6 wk after GBR-MSA with the application of UA-ADRCs, but not in GBR-MSA without UA-ADRCs, in most osteoblasts and osteoblast-like cells (arrows in Figure 8E). Only very weak anti-runt-related transcription factor 2 immunoreactivity was found at 34 wk after GBR-MSA (Figure 8G and 8H).

Type I collagen appeared in osteoid seams (arrows in Figure 8I and 8J) and in a weak manner in the matrix of newly formed bone trabeculae (asterisks in Figure 8I and 8J) at 6 wk after GBR-MSA. At 34 wk after GBR-MSA the pattern of immunoreactivity for type I collagen was similar to the pattern observed at 6 wk after GBR-MSA but weaker (arrows and asterisks in Figure 8K and 8L).

The application of UA-ADRCs resulted in strong alkaline phosphatase (AP) immunoreactivity in osteoblasts, osteoblast-like cells, and fibroblasts in the intertrabecular connective tissue at 6 wk after GBR-MSA, while in GBR-MSA without UA-ADRCs only a few osteoblasts were immunoreactive for AP (arrows in Figure 8M and 8N). At 34 wk after GBR-MSA AP immunoreactivity appeared in some osteoblasts and osteoblast-like cells in a weak to moderate manner (arrows in Figure 8O and 8P).

Immunohistochemical detection of factor VIII/von Willebrand factor showed dense vascularization in all biopsies (arrows in Figure 9A-D), with the highest density at 6 wk after GBR-MSA with the application of UA-ADRCs. In GBR-MSA without UA-ADRCs, larger sinusoidal vessels were found at 34 wk after GBR-MSA and were located within the intertrabecular connective tissue (arrowheads in Figure 9D). Vessel walls were strongly immunoreactive for CD146 in all biopsies (arrows in Figure 9E-H). A strong intracellular, granular anti-CD 73 immunoreactivity was seen in all biopsies in subsets of fibroblasts and osteoblasts (arrows in Figure 9I-L), as well as in vessel walls (arrowheads in Figure 9I-L). Nearly all osteoblasts and fibroblasts as well as a subset of osteocytes showed immunoreactivity for PPARγ at 6 wk after GBR-MSA (arrows in Figure 9M and 9N). Furthermore, PPARγ immunoreactivity was also found in vessel walls in areas with osteogenesis (arrowheads in Figure 9M and 9N). In general, a very similar pattern was found at 34 wk after the application of UA-ADRCs, whereas without UA-ADRCs immunoreactivity for PPARγ was restricted to vessel walls (arrows and arrowheads in Figure 9O and 9P). The immunohistochemically negative control specimens displayed no immunoreactivity.

### EXAMPLE SUMMARY

The study outlined in the examples is the first one in which a combination of freshly isolated UA-ADRCs, PRGF-2, and an OIS was used for GBR-MSA. Furthermore, said study is the first one in which cellular and histological effects of mesenchymal stem cells in human GBR-MSA were investigated with design-based stereology, histochemistry, and immunohistochemistry. The analysis of the biopsies that were collected at 6 wk and again at 34 wk after GBR-MSA showed that the combination of UA-ADRCs, PRGF-2, and the OIS resulted in better and faster bone regeneration than the combination of PRGF-2 and the same OIS alone. It is of note that said design-based stereologic finding of a higher relative amount of newly formed bone after GBR-MSA with the application of UA-ADRCs than without UA-ADRCs (+ 63% at 6 wk; + 44% at 34 wk) (Figure 7) was in line with the radiologic finding of more dense bone after the application of UA-ADRCs in regions 15 and 16 than without UA-ADRCs in regions 25 and 26 at 20 mo after GBR-MSA (Figure 4).

The results of the present study opens new horizons for the rehabilitation of the edentulous posterior maxilla and potentially for other regenerative techniques in pre-implant bone augmentation procedures like lateral and horizontal alveolar ridge augmentation, socket preservation, and bone grafting following large cystectomies. Specifically, the novel GBR-MSA approach presented here could result in a superior bone-implant-contact due to advanced new bone formation (Figure 7) and could also serve as the basis for reducing the time between GBR-MSA and the placement of implants in patients with residual bone height below the antrum and the ridge crest of less than 3 mm (Class D^{[32]}). Besides this, the present novel approach may allow for immediate implant placement in combination with bone augmentation procedures when the primary stability of the implant is provided. Moreover, the harvesting of ADRCs may result in less morbidity of the patient compared to bone marrow aspiration from the iliac crest area (addressed in the next paragraph).

GBR-MSA using mesenchymal stem cells (except of the use of UA-ADRCs) has been investigated in many preclinical and clinical studies, with and without OIS, and with and without use of PRP (reviewed in^{[62-64]}). In most of these studies BMSCs, autologous bone marrow aspirate concentrate (BMAC), or periosteal derived stem cells were used. Histomorphometric analysis showed considerable differences in the relative amount (area/area) of newly formed bone at 24 wk after surgery, ranging between 13.5% using BMSCs and bovine bone material (BBM)^{[65]} and 55.2% using BMAC and BBM^{[66]}. This considerable difference and the fact that the relative amount (area/area) of cancellous bone in normal human bone is only approximately 25%^{[67]} should give reason to accept the results of related studies only after having critically scrutinized the corresponding methodological details. For example, in a study that reported a relative amount of newly bone of 55.2% using BMAC and BBM^{[67]} only small portions of the investigated specimens were shown, and histomorphometry was performed by inspecting the specimens with a 1.25× objective, which precludes to distinguish between empty osteocyte lacunae (representing scaffold) and cell bodies within osteocyte lacunae (representing newly formed bone). In any case, a meta-analysis of nine studies (seven animal and two human studies) in which the combination of mesenchymal stem cells and OIS *versus* OIS alone were compared, found a statistically significant positive effect of stem cells on the bone re-growth in GBR-MSA^{[63]}.

At first glance our design-based results (relative amount of newly formed bone of 24.2% at 34 wk after GBR-MSA) do not speak in favor of our approach compared to the use of BMSCs (among the six studies reviewed in^{[63]} in which BMSCs were applied and histomorphometric analysis was performed at 24 wk after surgery, three studies reported a relative amount of newly formed bone of more than 24%). However, our decision to use UA-ADRCs rather than other types of cells (including ASCs, BMSCs, periosteal derived stem cells, allogeneic and/or modified ASCs/BMSCs, dental-derived mesenchymal stem cell-like cells (reviewed in^{[68]}) or induced pluripotent stem cells) was based on the fact that UA-ADRCs are the only type of cell that allows immediate usage at point of care, with the lowest safety concerns in cell-based therapy as no culturing or modification is required. This is fundamentally different from all other types of cells that have been considered for cell-based therapies in dentistry (reviewed in^{[69]}). Regarding safety concerns one must keep in mind that, e.g., potential oncogenesis currently limits the clinical translation of induced pluripotent stem cells^{[70,71]}, and applying allogeneic cells may cause the production of donor-specific antibodies and cell induced immune response^{[54,55]}. The only other cell-based therapy that allows immediate usage at point of care is autologous BMAC, which was investigated in two studies on GBR-MSA (BBM as OIS). One of these studies was a case report on a 46-year-old partially edentulous man, showing a relative amount of newly formed bone of 27% at 12 wk after surgery without control treatment^{[72]}. The other study^{[73]} was a randomized controlled trial (from the same lab as^{[72]}, published in the same year, and using exactly the same procedure for harvesting bone marrow aspirate as used in^{[72]}), reporting a mean relative amount of newly formed bone of only 12.6% at 12 wk after surgery (*n* = 34 patients), with a mean relative amount of newly formed bone of only 14.3% at 12 wk after grafting BBM and autologous bone (*n* = 11 patients)^{[73]}. It is of note that only in the latter study^{[73]} whole specimens were shown and methodological details of the histomorphometric analysis were provided. The data of the latter study^{[73]} are in line with the data obtained with our control treatment (Figure 7) and indicates that the use of UA-ADRCs may be more effective than the use of BMAC in GBR-MSA.

Detection of factors involved in osteogenesis and bone remodeling using histochemistry and immunohistochemistry is not frequently applied in studies on GBR-MSA in dentistry. Mostly, only selected factors were investigated. In line with former studies^{[74,75]} we applied a broader panel of antibodies including vessel markers like factor VIII/von Willebrand factor. The immunostaining pattern obtained revealed similar findings as for remodeling of allografts^{[76]}. However, the direct comparison of the immunolabeling of osteogenic factors like runt-related transcription factor 2 and AP between both sides showed stronger immunopositivity after the application of UA-ADRCs, which underlines better and faster bone regeneration. The finding of middle-sized osteoclasts on the surface of newly formed and allogeneic bone and on debris accumulations at 6 wk after GBR-MSA, with a higher osteoclast density after the application of UA-ADRCs than without UA-ADRCs (Figure 6E, 6F, and Figure 8A-D), was in line with earlier reports in the literature^{[77,78]} about osteoclasts involved in bone remodeling (which was increased after application of UA-ADRCs compared to the situation without UA-ADRCs). This phenomenon must not be confused with foreign material resorption by multinucleated giant cells^{[77]}. That was not observed in the present study. The denser vascularization on the cell treated side (detected with both histomorphometry and anti-factor VIII/von Willebrand factor immunohistochemistry) that was treated with UA-ARDCs underlines the importance of the coupling of angiogenesis and osteogenesis in bone regeneration^{[79]}.

We did not characterize the UA-ADRCs isolated from lipoaspirate with the Transpose RT system and Matrase Reagent (both from InGeneron) in the present study. However, a very recent study compared cell suspensions that were obtained by isolating cells from lipoaspirate from 12 healthy donors using the Transpose RT system and Matrase Reagent (thereafter: "TRT-MR cell suspensions") with cell suspensions that were obtained by isolating cells from lipoaspirate from the same donors just mechanically (*i.e.,* using the Transpose RT system but without Matrase Reagent) (thereafter: "TRT cell suspensions")^{[53]}. It was found that the mean cell yield (numbers of cells/g of processed lipoaspirate) was approximately twelve times higher in TRT-MR cell suspensions than in TRT cell suspensions (*P* < 0.001), and cells in TRT-MR cell suspensions formed on average 16 times more colony forming units (considered to be an indicator of stemness) per g lipoaspirate than cells in TRT cell suspensions (*P* < 0.001)^{[53]}. Of note, the mean relative number of viable cells in TRT-MR cell suspensions (85.9% ± 1.1%; mean ± SE) exceeded the proposed minimum threshold for the viability of cells in the stromal vascular fraction of 70% established by the International Federation for Adipose Therapeutics and Science^{[80]}, whereas the mean relative number of viable cells in TRT suspensions (61.7% ± 2.6%) did not (*P* < 0.001)^{[53]}. On the other hand, cells in TRT-MR cell suspensions exhibited no statistically significant differences in the expression of regenerative cell-associated genes such as Oct4, Hes1, and Klf4 compared to cells in TRT cell suspensions^{[53]}.

The same study demonstrated that upon stimulation with specific differentiation media cells in TRT-MR and TRT cell suspensions were independently able to differentiate into cells from all three germ layers (*i.e.,* into the adipogenic, osteogenic, hepatogenic, and neurogenic lineages)^{[53]}. The latter is in line with earlier findings that adult stem cells may obtain any of the lineages but depend on constant induction of differentiation and re-confirmation by signals released and communicated from the local microenvironment^{[81-83]}. If this information and confirmation is missing or ceases, adult stem cells stop differentiating^{[84,85]}. In fact, only true stem cells are able to continue their expected differentiation pathway as supported by the local microenvironment^{[86-88]}. This is one of several reasons why adult stem cell therapy with UA-ARDCs is very safe^{[89-91]}. In contrast, safety concerns have been raised regarding stem cell therapy with cultured adult stem cells because with higher passages an increased rate of potential malignant transformation may occur^{[92-94]}.

A study on fresh, uncultured cells that were isolated from adipose tissue of pigs using the Transpose RT system and Matrase Reagent showed that approximately 40% of cells in the stromal vascular fraction were immunopositive for CD29 (thereafter: CD29⁺) and CD44^{+[95]}, which are markers of adipose tissue-derived stem cells^{[50,96]}. Furthermore, on average only 9% of the cells were CD45⁺ (a marker of blood derived cells^{[50]}), and on average only 11% of the cells were CD31⁺ (a marker of endothelial cells^{[50]}). Another study on fresh, uncultured cells that were isolated from adipose tissue of horses using a predecessor of the Transpose RT system (ARC system; InGeneron) and Matrase Reagent found the highest relative gene expression of osteocalcin (a gene associated with the osteogenic lineage^{[97]}) when investigating these cells for the relative gene expression of CD44, CD73, CD90, CD105, CD146, and CD166 (mesenchymal stem cell surface markers), CD34 and CD45 (hematopoietic markers), CD31 (endothelial cell marker), type-1 collagen, PPARγ2 (a gene associated with the adipogenic lineage), and osteocalcin^{[98]}. Collectively, these data underline the osteogenic potential of the UA-ADRCs used in the present study.

We used PRGF-2 rather than PRP because our clinical experience is in line with data from a recent experimental study on rats that showed that PRGF-2 has more availability for bone regeneration than PRP^{[34]}. The content of PRGF-2 prepared using the PRGF-Endoret technology (BTI) was investigated in several studies in the literature. Most relevant to the results of the present study was the demonstration of high amounts of growth factors in PRGF-2^{[98]}, *i.e.* on average approximately 14000 pg/mL of platelet derived growth factor-AB, 46000 pg/mL of transforming growth factor-β 1, 220 pg/mL of vascular endothelial growth factor, 400 pg/mL hepatocyte growth factor, 83000 pg/mL insulin-like growth factor-1, and 600 pg/mL endothelial growth factor (note that what was named "PRGF F3" in^{[98]} is now named "Fraction 2 of PRGF" according to BTI, and the latter terminology was used in the present study). Several pilot studies described the use of PRGF in GBR-MSA^{[99-101]}. Furthermore, it was shown that PRGF can stimulate the proliferation, migration, and chemotaxis of osteoblasts *in vitro* and enhance the osteblasts' autocrine expression of vascular endothelial growth factor and hepatocyte growth factor that are proangiogenic factors^{[98]}. Both vascular endothelial growth factor and hepatocyte growth factor are contained in PRGF-2^{[98]} and were also identified within the ASCs' secretome (reviewed in^{[102]}). A recent study showed that PRGF can induce proliferation and migration of human-derived ASCs and reduce senescence and autophagocytosis of these cells *in vitro*^{[103]}*.* The same study^{[103]} confirmed our own finding that human-derived ASCs can efficiently differentiate into osteocytes or adipocytes when cultured in osteogenic or adipogenic induction medium, respectively^{[53,104]}, but also demonstrated that this process is enhanced in the presence of PRGF^{[103]}. Accordingly, our finding that the treatment with MCBPA/PRGF-2/UA-ADRCs resulted in the formation of 44% more bone and 83% fewer adipocytes in the biopsies collected at 34 wk after GBR-MSA (Figure 7) cannot be directly attributed to the action of PRGF-2 on UA-ADRCs. Rather, it is reasonable to hypothesize that cues from the local extracellular environment affected the properties of the UA-ADRCs in terms of proliferation and specific osteogenic differentiation (c.f.^{[105]}), and this process was enhanced by PRGF-2.

GBR-MSA has been performed using a number of grafting materials, including autologous bone grafts, allografts, and xenografts^{[106]}. An earlier meta-analysis published in 1998 found that the survival rates of implants placed in grafted maxillary sinuses did not depend on whether autologous, allogeneic, or alloplastic grafts were used^{[107]}. However, when focusing on the total bone volume, another meta-analysis published in 2010 concluded that autologous bone should still be considered the gold standard in MSA surgery^{[108]}. On the other hand, the latter study stated that the consequence of the total bone volume for implant survival is still unknown^{[108]}. A recent meta-analysis based on 16 original studies found that the implant survival rate was 99.6% when a biomaterial was used during surgery compared to 96.0% when no graft material was used (the follow-up period was 48 to 60 mo in this study)^{[109]}. However, these data should be handled cautiously because only two studies in this meta-analysis were performed with autologous bone grafts, and in 10 out of the 16 included studies (six out of seven studies without interpositional graft material) the average preoperative bone height was more than 5 mm, which represents Class C according to ^{[32]} and does not resemble the situation addressed in the present study. We used the Puros Cancellous Particulate Allograft (Zimmer Biomet Dental) because it fulfills all of the following criteria that are considered essential for an OIS in the literature (reviewed in^{[110]}: volume stability of at least 4 mo after implantation, full biocompatibility and resorbability, and possibility for loading with stem cells and growth factors.

The patient who was investigated in our study was 79-years-old. The statistical life expectancy of 80-year-old men and women has increased 7.7 years and 9.2 years, respectively in Germany^{[111]}. Thus, one can expect an increasing demand for the restoration of an aesthetic smile and a functional occlusion by patients aged 79 and older (and of course by younger patients) in the future. On the other hand, some authors have argued that age-related progressive decline in mechanical strength of tissue could be due to loss of resident stem cell number and function and have raised concerns regarding the use of autologous adult stem cell therapy in older patients^{[112]}. Indeed, an earlier study^{[113]} found that the number and multilineage differentiation potential of ASCs declined with the age of healthy volunteers, combined with increased expression of cyclin-dependent kinase inhibitor p16ink4a and CHEK1 (*i.e.,* genes associated with senescence)^{[112]}. These and other data reported in the literature have motivated patients to start cryopreserving ADRCs from lipoaspirate^{[112]}. However, a recent pilot study showed that two samples of ADRCs collected from a healthy person at age 72 years and again at age 84 years showed the same cell yield and ADRC subpopulation composition without change in the proliferation rates of ASCs (obtained by culturing the ADRCs), as well as the capability of tri-lineage differentiation of both cell cultures^{[112]}. Another recent pilot study showed that protein expression profiles of human umbilical vein endothelial cells that were co-cultured under oxidative stress conditions with ADRCs from three healthy persons aged 42, 45, and 47 years did not differ from protein expression profiles of human umbilical vein endothelial cells that were co-cultured under identical conditions with ADRCs from three healthy persons aged 61 and 62 (two persons) years^{[114]}. Collectively, these data with the results of the present study, indicate (albeit preliminary) that using freshly isolated UA-ADRCs from patients aged 79 years and older is a valid approach for GBR-MSA. Effects of aging on PRGF-2 have, to our knowledge, not been reported.

### REFERENCES

1 Laudenbach JM, Simon Z. Med Clin North Am 2014; 98: 1239-1260
2 Moraschini V, Poubel LA, Ferreira VF, Barboza Edos S. Int J Oral Maxillofac Surg 2015; 44: 377-388
3 Lundgren S, Cricchio G, Hallman M, Jungner M, Rasmusson L, Sennerby L. Periodontol 2000 2017; 73: 103-120
4 Esposito M, Felice P, Worthington HV. Cochrane Database Syst Rev 2014; (5) : CD008397
5 Starch-Jensen T, Jensen JD. J Oral Maxillofac Res 2017; 8: e3
6 Al-Dajani M. Clin Implant Dent Relat Res 2016; 18: 204-212
7 Jamjoom A, Cohen RE. J Funct Biomater 2015; 6: 833-848
8 Rogers GF, Greene AK. J Craniofac Surg 2012; 23: 323-327
9 Sakkas A, Wilde F, Heufelder M, Winter K, Schramm A. Int J Implant Dent 2017; 3**:**
10 Sanz M, Vignoletti F. Dent Mater 2015; 31: 640-647
11 Wang W, Yeung KWK. Bioact Mater 2017; 2: 224-247
12 Yamada M, Egusa H. J Prosthodont Res 2018; 62: 152-161
13 Nkenke E, Neukam FW. Eur J Oral Implantol 2014; 7 Suppl 2: S203-S217
14 Garcia-Gareta E, Coathup MJ, Blunn GW. Bone 2015; 81: 112-121
15 Troeltzsch M, Troeltzsch M, Kauffmann P, Gruber R, Brockmeyer P, Moser N, Rau A, Schliephake H. J Craniomaxillofac Surg 2016; 44: 1618-1629
16 Danesh-Sani SA, Engebretson SP, Janal MN. J Periodontal Res 2017; 52: 301-312
17 Monje A, O'Valle F, Monje-Gil F, Ortega-Oller I, Mesa F, Wang HL, Galindo-Moreno P. Int J Oral Maxillofac Implants 2017; 32: 121-127
18 Wood RA, Mealey BL. J Periodontol 2012; 83: 329-336
19 Miron RJ, Sculean A, Shuang Y, Bosshardt DD, Gruber R, Buser D, Chandad F, Zhang Y. Clin Oral Implants Res 2016; 27: 668-675
20 Roldán JC, Knueppel H, Schmidt C, Jepsen S, Zimmermann C, Terheyden H. Clin Oral Implants Res 2008; 19: 373-378
21 Lee HJ, Choi BH, Jung JH, Zhu SJ, Lee SH, Huh JY, You TM, Li J. Oral Surg Oral Med Oral Pathol Oral Radiol Endod 2007; 103: 329-333
22 Kassolis JD, Rosen PS, Reynolds MA. J Periodontol 2000; 71: 1654-1661
23 Rodriguez A, Anastassov GE, Lee H, Buchbinder D, Wettan H. J Oral Maxillofac Surg 2003; 61: 157-163
24 Wiltfang J, Schlegel KA, Schultze-Mosgau S, Nkenke E, Zimmermann R, Kessler P. Clin Oral Implants Res 2003; 14: 213-218
25 Bae JH, Kim YK, Myung SK. J Periodontol 2011; 82: 660-667
26 Nikolidakis D, Jansen JA. Tissue Eng Part B Rev 2008; 14: 249-258
27 Intini G. Biomaterials 2009; 30: 4956-4966
28 Roffi A, Di Matteo B, Krishnakumar GS, Kon E, Filardo G. Int Orthop 2017; 41: 221-237
29 Lemos CA, Mello CC, dos Santos DM, Verri FR, Goiato MC, Pellizzer EP. Int J Oral Maxillofac Surg 2016; 45: 517-525
30 Pocaterra A, Caruso S, Bernardi S, Scagnoli L, Continenza MA, Gatto R. Int J Oral Maxillofac Surg 2016; 45: 1027-1034
31 Abdalla RIB, Alqutaibi AY, Kaddah A. Quintessence Int 2018; 49: 139-146
32 Misch CE. Available bone and dental implant treatment plans. In: Misch CE, editor. Contemporary Implant Dentistry. 3rd ed. St. Louis: Mosby, 2008: 178-199
33 Jensen OT, Shulman LB, Block MS, Iacono VJ. Int J Oral Maxillofac Implants 1998; 13 Suppl: 11-45
34 Eda T, Takahashi K, Kanao S, Aoki A, Ogura N, Ito K, Tsukahara H, Suemitsu M, Kuyama K, Kondoh T. J Oral Maxillofac Surg Med Pathol 2017; 29: 563-569
35 Parnia F, Yazdani J, Maleki Dizaj S. Stem Cells Int 2018; 2018: 3080139
36 Niño-Sandoval TC, Vasconcelos BC, D Moraes SL, A Lemos CA, Pellizzer EP. Int J Oral Maxillofac Surg 2018
37 Miguita L, Mantesso A, Pannuti CM, Deboni MCZ. Cell Tissue Bank 2017; 18: 217-228
38 Barba M, Di Taranto G, Lattanzi W. Expert Opin Biol Ther 2017; 17: 677-689
39 Bhattacharya I, Ghayor C, Weber FE. Transfus Med Hemother 2016; 43: 336-343
40 Han DS, Chang HK, Kim KR, Woo SM. J Craniofac Surg 2014; 25: 196-201
41 Hayashi O, Katsube Y, Hirose M, Ohgushi H, Ito H. Calcif Tissue Int 2008; 82: 238-247
42 Zhang W, Zhang X, Wang S, Xu L, Zhang M, Wang G, Jin Y, Zhang X, Jiang X. J Dent Res 2013; 92: 1136-1141
43 Yu H, Lu K, Zhu J, Wang J. Br Med Bull 2017; 121: 135-154
44 Fraser JK, Wulur I, Alfonso Z, Hedrick MH. Trends Biotechnol 2006; 24: 150-154
45 Aust L, Devlin B, Foster SJ, Halvorsen YD, Hicok K, du Laney T, Sen A, Willingmyre GD, Gimble JM. Cytotherapy 2004; 6: 7-14
46 Tajima S, Tobita M, Mizuno H. Histol Histopathol 2018; 33: 619-627
47 Romagnoli C, Brandi ML. World J Stem Cells 2014; 6: 144-152
48 Zanetti AS, Sabliov C, Gimble JM, Hayes DJ. J Biomed Mater Res B Appl Biomater 2013; 101: 187-199
49 Oberbauer E, Steffenhagen C, Wurzer C, Gabriel C, Redl H, Wolbank S. Cell Regen (Lond) 2015; 4: 7
50 van Dongen JA, Tuin AJ, Spiekman M, Jansma J, van der Lei B, Harmsen MC. J Tissue Eng Regen Med 2018; 12: e261-e274
51 Condé-Green A, Kotamarti VS, Sherman LS, Keith JD, Lee ES, Granick MS, Rameshwar P. Plast Reconstr Surg Glob Open 2016; 4: e1017
52 Aronowitz JA, Lockhart RA, Hakakian CS. Springerplus 2015; 4: 713
53 Valenzuela N, Alt C, Winnier GE, Alt EU. Isolation of adipose tissue derived regenerative cells from human subcutaneous tissue with or without the use of enzymatic reagent. 2018 Preprint. Available from: bioRxiv:485318
54 Panes J, Garcia-Olmo D, Van Assche G, Colombel JF, Reinisch W, Baumgart DC, Dignass A, Nachury M, Ferrante M, Kazemi-Shirazi L, Grimaud JC, de la Portilla F, Goldin E, Richard MP, Leselbaum A, Danese S; ADMIRE CD Lancet 2016; 388: 1281-1290
55 Álvaro-Gracia JM, Jover JA, García-Vicuña R, Carreño L, Alonso A, Marsal S, Blanco F, Martinez-Taboada VM, Taylor P, Martin-Martin C, DelaRosa O, Tagarro I, Diaz-Gonzalez F. Ann Rheum Dis 2017; 76: 196-202
56 Schmitz C, Hof PR. Neuroscience 2005; 130: 813-831
57 Klein JA. Plast Reconstr Surg 1993; 92: 1085-98; discussion 1099-100
58 Coleman WP 4th, Hendry SL 2nd. Semin Cutan Med Surg 2006; 25: 138-144
59 Tatum H Jr. Dent Clin North Am 1986; 30: 207-229
60 El Chaar ES. Implant Dent 2010; 19: 370-377
61 Ronda M, Stacchi C. Int J Periodontics Restorative Dent 2015; 35: 795-801
62 Mangano FG, Tettamanti L, Sammons RL, Azzi L, Caprioglio A, Macchi A, Mangano C. Oral Surg Oral Med Oral Pathol Oral Radiol 2013; 115: 717-723
63 Mangano FG, Colombo M, Veronesi G, Caprioglio A, Mangano C. World J Stem Cells 2015; 7: 976-991
64 Correia F, Pozza DH, Gouveia S, Felino A, Faria E Almeida R. Clin Implant Dent Relat Res 2018; 20: 229-242
65 Wildburger A, Payer M, Jakse N, Strunk D, Etchard-Liechtenstein N, Sauerbier S. Clin Oral Implants Res 2014; 25: 1175-1181
66 Pasquali PJ, Teixeira ML, de Oliveira TA, de Macedo LG, Aloise AC, Pelegrine AA. Int J Biomater 2015; 2015: 121286
67 Parisien M, Cosman F, Morgan D, Schnitzer M, Liang X, Nieves J, Forese L, Luckey M, Meier D, Shen V, Lindsay R, Dempster DW. J Bone Miner Res 1997; 12: 948-957
68 Hynes K, Menicanin D, Gronthos S, Bartold PM. Periodontol 2000 2012; 59: 203-227
69 Egusa H, Sonoyama W, Nishimura M, Atsuta I, Akiyama K. J Prosthodont Res 2012; 56: 151-165
70 Ahmed RP, Ashraf M, Buccini S, Shujia J, Haider HKh. Regen Med 2011; 6: 171-178
71 Zhang Y, Wang D, Chen M, Yang B, Zhang F, Cao K. PLoS One 2011; 6: e19012
72 Schmelzeisen R, Gutwald R, Oshima T, Nagursky H, Vogeler M, Sauerbier S. Br J Oral Maxillofac Surg 2011; 49: 480-482
73 Sauerbier S, Rickert D, Gutwald R, Nagursky H, Oshima T, Xavier SP, Christmann J, Kurz P, Menne D, Vissink A, Raghoebar G, Schmelzeisen R, Wagner W, Koch FP. Tissue Eng Part A 2011; 17: 2187-2197
74 Götz W, Gerber T, Michel B, Lossdörfer S, Henkel KO, Heinemann F. Clin Oral Implants Res 2008; 19: 1016-1026
75 Friedmann A, Gissel K, Konermann A, Götz W. Clin Oral Investig 2015; 19: 1595-1603
76 Hawthorne AC, Xavier SP, Okamoto R, Salvador SL, Antunes AA, Salata LA. Clin Oral Implants Res 2013; 24: 1164-1172
77 Brunel G, Brocard D, Duffort JF, Jacquet E, Justumus P, Simonet T, Benqué E. J Periodontol 2001; 72: 257-264
78 Mordenfeld A, Hallman M, Johansson CB, Albrektsson T. Clin Oral Implants Res 2010; 21: 961-970
79 Tian T, Zhang T, Lin Y, Cai X. J Dent Res 2018; 97: 969-976
80 Bourin P, Bunnell BA, Casteilla L, Dominici M, Katz AJ, March KL, Redl H, Rubin JP, Yoshimura K, Gimble JM. Cytotherapy 2013; 15: 641-648
81 Rezza A, Sennett R, Rendl M. Curr Top Dev Biol 2014; 107: 333-372
82 Dong L, Hao H, Han W, Fu X. Sci China Life Sci 2015; 58: 639-648
83 Wabik A, Jones PH. EMBO J 2015; 34: 1164-1179
84 Trosko JE. Anat Rec (Hoboken) 2014; 297: 161-173
85 Zhang B, Wang M, Gong A, Zhang X, Wu X, Zhu Y, Shi H, Wu L, Zhu W, Qian H, Xu W. Stem Cells 2015; 33: 2158-2168
86 Phinney DG, Prockop DJ. Stem Cells 2007; 25: 2896-2902
87 Kara RJ, Bolli P, Karakikes I, Matsunaga I, Tripodi J, Tanweer O, Altman P, Shachter NS, Nakano A, Najfeld V, Chaudhry HW. Circ Res 2012; 110: 82-93
88 Malliaras K, Zhang Y, Seinfeld J, Galang G, Tseliou E, Cheng K, Sun B, Aminzadeh M, Marbán E. EMBO Mol Med 2013; 5: 191-209
89 Ra JC, Shin IS, Kim SH, Kang SK, Kang BC, Lee HY, Kim YJ, Jo JY, Yoon EJ, Choi HJ, Kwon E. Stem Cells Dev 2011; 20: 1297-1308
90 Hoffman AM, Dow SW. Stem Cells 2016; 34: 1709-1729
91 Toyserkani NM, Jørgensen MG, Tabatabaeifar S, Jensen CH, Sheikh SP, Sørensen JA. Stem Cells Transl Med 2017; 6: 1786-1794
92 Røsland GV, Svendsen A, Torsvik A, Sobala E, McCormack E, Immervoll H, Mysliwietz J, Tonn JC, Goldbrunner R, Lønning PE, Bjerkvig R, Schichor C. Cancer Res 2009; 69: 5331-5339
93 Xu S, De Becker A, De Raeve H, Van Camp B, Vanderkerken K, Van Riet I. Biochem Biophys Res Commun 2012; 424: 391-397
94 Pan Q, Fouraschen SM, de Ruiter PE, Dinjens WN, Kwekkeboom J, Tilanus HW, van der Laan LJ. Exp Biol Med (Maywood) 2014; 239: 105-115
95 Haenel A, Ghosn M, Karimi T, Vykoukal J, Kettlun C, Shah D, Dave A, Valderrrabano M, Schulz D, Azares A, Raizner A, Alt E. Unmodified, autologous adipose-derived regenerative cells improve cardiac function, structure and revascularization in a porcine model of chronic myocardial infarction. 2018 Preprint. BioRxiv:286468
96 Badimon L, Oñate B, Vilahur G. Rev Esp Cardiol (Engl Ed) 2015; 68: 599-611
97 Lombardi G, Perego S, Luzi L, Banfi G. Endocrine 2015; 48: 394-404
98 Anitua E, Tejero R, Zalduendo MM, Orive G. J Periodontol 2013; 84: 1180-1190
99 Anitua E, Prado R, Orive G. Clin Implant Dent Relat Res 2009; 11 Suppl 1: e23-e31
100 Taschieri S, Corbella S, Del Fabbro M. J Oral Implantol 2012; 38: 621-627
101 Khouly I, Pardiñas López S, Aliaga I, Froum SJ. Implant Dent 2017; 26: 199-208
102 Salgado AJ, Reis RL, Sousa NJ, Gimble JM. Curr Stem Cell Res Ther 2010; 5: 103-110
103 Mellado-López M, Griffeth RJ, Meseguer-Ripolles J, Cugat R, Garcia M, Moreno-Manzano V. Stem Cells Int 2017; 2017: 5946527
104 Bai X, Sadat S, Gehmert S, Alt E, Song YH. FEBS Lett 2007; 581: 4681-4684
105 Kusuma GD, Carthew J, Lim R, Frith JE. Stem Cells Dev 2017; 26: 617-631
106 Hegde R, Prasad K, Shroff KK. J Indian Prosthodont Soc 2016; 16: 317-322
107 Tong DC, Rioux K, Drangsholt M, Beirne OR. Int J Oral Maxillofac Implants 1998; 13: 175-182
108 Klijn RJ, Meijer GJ, Bronkhorst EM, Jansen JA. Tissue Eng Part B Rev 2010; 16: 493-507
109 Silva LD, de Lima VN, Faverani LP, de Mendonça MR, Okamoto R, Pellizzer EP. Int J Oral Maxillofac Surg 2016; 45: 1570-1576
110 Hosseinpour S, Ghazizadeh Ahsaie M, Rezai Rad M, Baghani MT, Motamedian SR, Khojasteh A. Oral Maxillofac Surg 2017; 21: 109-129
111 Destatis Statistisches Bundesamt. 2018. Available from: URL: https://www.destatis.de/DE/ZahlenFakten/GesellschaftStaat/Bevoelkerung/Sterbefaelle/Tabellen/Lebenserwartung.pdf
112 Kokai LE, Traktuev DO, Zhang L, Merfeld-Clauss S, DiBernardo G, Lu H, Marra KG, Donnenberg A, Donnenberg V, Meyer EM, Fodor PB, March KL, Rubin JP. Aesthet Surg J 2017; 37: 454-463
113 Alt EU, Senst C, Murthy SN, Slakey DP, Dupin CL, Chaffin AE, Kadowitz PJ, Izadpanah R. Stem Cell Res 2012; 8: 215-225
114 Kim SW, Choi JW, Lee CY, Lee J, Shin S, Lim S, Lee S, Kim IK, Lee HB, Hwang KC. J Int Med Res 2018; 46: 951-964
115 Egusa H, Sonoyama W, Nishimura M, Atsuta I, Akiyama K. J Prosthodont Res 2012; 56: 229-248
116 Miran S, Mitsiadis TA, Pagella P. Stem Cells Int 2016; 2016: 7231038
117 Amrollahi P, Shah B, Seifi A, Tayebi L. Mater Sci Eng C Mater Biol Appl 2016; 69: 1383-1390

## Claims

1. Kit comprising
i) unmodified autologous adipose-derived regenerative cells (UA-ADRCs), wherein said UA-ADRCs are isolated from adipose tissue comprising collagenase digestion of said tissue, followed by centrifugal density gradient separation,
ii) an autologous fraction 2 of plasma rich in growth factors (PRGF-2), and
iii) an osteoinductive scaffold (OIS).

2. The kit of claim 1, wherein said UA-ADRCs are non-cultured UA-ADRCs and are isolated from in-vivo human adipose tissue.

3. The kit of claim 2, wherein said UA-ADRCs are isolated from a lipoaspirate.

4. The kit of claim 3, wherein said lipoaspirate is a liposuction aspirate.

5. The kit of any one of claims 2 to 4, wherein said UA-ADRCs are freshly isolated UA-ADRCs or cryopreserved UA-ADRCs.

6. The kit of claim 5, wherein said UA-ADRCs are isolated from a cryopreserved lipoaspirate or a freshly non-cryopreserved lipoaspirate immediately following its harvesting from the human.

7. The kit of any one of claims 1-6 for use as a medicament.

8. The kit for use of claim 7, wherein said kit is for use in bone augmentation procedures (BAP) and/or guided bone regeneration (GBR).

9. The kit for use of claim 8, wherein said kit is for use in
a) pre-implant BAP and/or GBR,
b) immediate implant placement, or
c) pre-implant BAP and/or GBR in combination with its use in implant placement.

10. The kit for use of any one of claims 7 to 9, wherein said use is in oral surgery.

11. The kit for use of any one of claims 7 to 10, wherein said use is for pre-implant bone augmentation selected from the group consisting of lateral and horizontal alveolar ridge augmentation, socket preservation, bone grafting following cystectomies and rehabilitation of the edentulous posterior maxilla.

12. The kit for use of claim 11, wherein said use is for rehabilitation of the edentulous posterior maxilla.

13. The kit for use of any one of claims 7 to 10, wherein said use comprises a bone preparation for implant placement.

14. The kit for use of claim 13, wherein said use comprises maxillary sinus augmentation (MSA) and/or lateral ridge augmentation (LRA) prior to implant placement.

## Patentansprüche

1. Kit umfassend
i) unmodifizierte autologe, aus Fettgewebe stammende regenerative Zellen (UA-ADRCs), wobei die UA-ADRCs aus Fettgewebe isoliert werden, umfassend einen Kollagenase-Verdau des Gewebes, gefolgt von einer zentrifugalen Dichtegradiententrennung,
ii) eine autologe Fraktion 2 aus wachstumsfaktorreichem Plasma (PRGF-2), und
iii) ein osteoinduktives Gerüst (OIS).

2. Kit nach Anspruch 1, wobei die UA-ADRCs nicht kultivierte UA-ADRCs sind und aus menschlichem in-vivo-Fettgewebe isoliert werden.

3. Kit nach Anspruch 2, wobei die UA-ADRCs aus einem Lipoaspirat isoliert sind.

4. Kit nach Anspruch 3, wobei das Lipoaspirat ein Liposuktionsaspirat ist.

5. Kit nach einem der Ansprüche 2 bis 4, wobei die UA-ADRCs frisch isolierte UA-ADRCs oder kryokonservierte UA-ADRCs sind.

6. Kit nach Anspruch 5, wobei die UA-ADRCs aus einem kryokonservierten Lipoaspirat oder einem frischen, nicht kryokonservierten Lipoaspirat unmittelbar nach seiner Entnahme aus dem Menschen isoliert werden.

7. Kit nach einem der Ansprüche 1-6 zur Verwendung als Medikament.

8. Kit zur Verwendung nach Anspruch 7, wobei das Kit zur Verwendung bei Knochenaufbauverfahren (BAP) und/oder gesteuerter Knochenregeneration (GBR) bestimmt ist.

9. Kit zur Verwendung nach Anspruch 8, wobei das Kit zur Verwendung in folgenden Fällen bestimmt ist
a) präimplantäres BAP und/oder GBR
b) sofortige Implantatplazierung, oder
c) präimplantäres BAP und/oder GBR in Kombination mit der Verwendung bei der Implantatplazierung.

10. Kit zur Verwendung nach einem der Ansprüche 7 bis 9, wobei die Verwendung in der Oralchirurgie erfolgt.

11. Kit zur Verwendung nach einem der Ansprüche 7 bis 10, wobei die Verwendung zur präimplantären Knochenaugmentation erfolgt, ausgewählt aus der Gruppe bestehend aus lateraler und horizontaler Alveolarkammaugmentation, Schaftkonservierung, Knochentransplantation nach Zystektomien und Sanierung des zahnlosen hinteren Oberkiefers.

12. Kit zur Verwendung nach Anspruch 11, wobei die Verwendung zur Sanierung des zahnlosen hinteren Oberkiefers dient.

13. Kit zur Verwendung nach einem der Ansprüche 7 bis 10, wobei die Verwendung eine Knochenpräparation für die Implantatplazierung umfasst.

14. Kit zur Verwendung nach Anspruch 13, wobei die Verwendung eine maxillare Sinus-Augmentation (MSA) und/oder eine laterale Kamm-Augmentation (LRA) vor der Implantatplatzierung umfasst.

## Revendications

1. Kit comprenant
i) des cellules régénératives non modifiées issues du tissu adipeux (UA-ADRC), dans lequel lesdites UA-ADRC sont isolées d'un tissu adipeux comprenant une digestion dudit tissu par la collagénase, suivie d'une séparation centrifuge par gradient de densité,
ii) une fraction autologue 2 de plasma riche en facteurs de croissance (PRGF-2), et
iii) un échafaudage ostéo-inductif (OIS).

2. Kit selon la revendication 1, dans lequel lesdites UA-ADRC sont des UA-ADRC non cultivées et sont isolées d'un tissu adipeux humain in vivo.

3. Kit selon la revendication 2, dans lequel lesdites UA-ADRC sont isolées d'un lipoaspirat.

4. Kit selon la revendication 3, dans lequel ledit lipoaspirat est un aspirat de liposuccion.

5. Kit selon l'une quelconque des revendications 2 à 4, dans lequel lesdites UA-ADRC sont des UA-ADRC fraîchement isolées ou des UA-ADRC cryoconservées.

6. Kit selon la revendication 5, dans lequel lesdites UA-ADRC sont isolées d'un lipoaspirat cryoconservé ou d'un lipoaspirat fraîchement non cryoconservé, immédiatement après son prélèvement à partir de l'humain.

7. Kit selon l'une quelconque des revendications 1 à 6 pour une utilisation comme médicament.

8. Kit pour une utilisation selon la revendication 7, ledit kit étant destiné à une utilisation dans des techniques d'augmentation osseuse (BAP) et/ou une régénération osseuse guidée (ROG).

9. Kit pour une utilisation selon la revendication 8, ledit kit étant destiné à une utilisation dans
a) une BAP et/ou une ROG pré-implantation,
b) un placement implantaire immédiat, ou
c) une BAP et/ou une ROG pré-implantation, en combinaison avec son utilisation en placement implantaire.

10. Kit pour une utilisation selon l'une quelconque des revendications 7 à 9, dans lequel ladite utilisation s'effectue en chirurgie orale.

11. Kit pour une utilisation selon l'une quelconque des revendications 7 à 10, dans lequel ladite utilisation est destinée à une augmentation osseuse pré-implantaire choisie dans le groupe consistant en une augmentation du bord alvéolaire latéral et horizontal, une conservation alvéolaire, un greffage osseux après des cystectomies et une réhabilitation de la mandibule postérieure édentée.

12. Kit pour une utilisation selon la revendication 11, dans lequel ladite utilisation est destinée à une réhabilitation de la mandibule postérieure édentée.

13. Kit pour une utilisation selon l'une quelconque des revendications 7 à 10, dans lequel ladite utilisation comprend une préparation osseuse pour placement implantaire.

14. Kit pour une utilisation selon la revendication 13, dans lequel ladite utilisation comprend une augmentation du sinus maxillaire (MSA) et/ou une augmentation du bord latéral (LRA) avant placement implantaire.
